# EUROPÄISCHE PATENTSCHRIFT

(11) **EP 2 544 751 B1**
(45) Veröffentlichungstag und Bekanntmachung des Hinweises auf die Patenterteilung: **01.07.2020**
(21) Anmeldenummer: 11718892.0
(22) Anmeldetag: 28.02.2011
(51) Int. Cl.: A61M 25/09, A61B 1/018

(54) **FÜHRUNGSDRAHTHALTER ZUM AUFNEHMEN UND FESTHALTEN EINES MEDIZINISCHEN FÜHRUNGSDRAHTES UND ZUM ANBRINGEN AN EINEM MEDIZINISCHEN GERÄT, INSBESONDERE AN EINEM ENDOSKOP**
GUIDE WIRE HOLDER FOR RECEIVING AND SECURING A MEDICAL GUIDE WIRE AND FOR MOUNTING ON A MEDICAL APPLIANCE, IN PARTICULAR ON AN ENDOSCOPE
PORTE-CÂBLE DE GUIDAGE POUR LA RÉCEPTION ET LE MAINTIEN FERME D'UN CÂBLE DE GUIDAGE MÉDICAL ET POUR LA POSE SUR UN APPAREIL MÉDICAL, EN PARTICULIER SUR UN ENDOSCOPE

(30) Priorität: 12.03.2010 DE 102010011222
(43) Veröffentlichungstag der Anmeldung: 16.01.2013
(73) Patentinhaber: Medi-Globe GmbH, 83101 Rohrdorf-Achenmühle (DE)
(72) Erfinder: EDEN, Ingo, 83101 Rohrdorf-Achenmühle (DE)
(74) Vertreter: Hofstetter, Schurack & Partner
(86) Internationale Anmeldenummer: PCT/DE2011/000180
(87) Internationale Veröffentlichungsnummer: WO 2011/110152

(56) Entgegenhaltungen:
- US-A- 4 844 092
- US-A1- 2004 162 465
- US-A1- 2006 195 117

## Beschreibung

Die Erfindung bezieht sich auf einen Führungsdrahthalter zum Aufnehmen und Festhalten eines medizinischen Führungsdrahtes und zum Anbringen an einem medizinischen Gerät, insbesondere an einem Endoskop, mit einem Anbringungsteil, durch welches der Führungsdraht hindurchführbar ist, und einem mit dem Anbringungsteil verbundenen und von diesem abstehenden Führungsdrahtträger, der den Führungsdraht mittels einer Führungsdraht-Aufnahmeeinrichtung festzuhalten gestattet, die ein den Führungsdraht in Abstand von dem Anbringungsteil in eine von seiner Durchführungsrichtung in dem Anbringungsteil wegführende Richtung umleitendes und spannendes Führungsdraht-Umlenkglied enthält, an das sich in weiteren Abständen von dem Anbringungsteil ein zusätzliches Führungsdraht-Umlenkelement und ein noch weiteres Führungsdraht-Umlenkelement anschließen.

Ein Führungsdrahthalter der vorstehenden Art ist bereits bekannt (US 2006/195117 A1; WO 2007/086876 A2).

Nach dem bisher bekannten Stand der Technik gibt es für das Festhalten eines Führungsdrahtes zwei prinzipiell unterschiedliche Lösungen. Gemäß der einen Lösung wird die Reibung an einer Mehrzahl von gegebenenfalls kantige Umlenkflächen aufweisenden Vorsprüngen ausgenutzt, die von einem mit einem Anbringungsteil verbundenen Trägerteil seitlich abstehen und um die der Führungsdraht gefädelt wird. Gemäß der anderen Lösung wird die Reibung an Kanten von Öffnungen ausgenutzt, die sich in einer Halteplatte befinden, welche mittels eines Anbringungsteiles an einem medizinischen Gerät anbringbar ist.

Zu der erstgenannten Lösung gehört ein Führungsdrahthalter, der zur Befestigung eines länglichen medizinischen Geräts, nämlich eines Führungsdrahtes an einer länglichen Einführvorrichtung, insbesondere an einem Endoskop dient (US 7 637 863 B2; EP 1 654 026 B1). Dabei weist der Führungsdrahthalter ein von einem Anbringungsteil zu seinem Anbringen an dem Endoskop weg laufendes Rippenteil auf, an dem mehrere, zumindest drei nach außen sich erstreckende Vorsprünge vorgesehen sind, um die der Führungsdraht gefädelt wird. Dabei liegen die mehreren Vorsprünge auf gegenüberliegenden Seiten des Führungsdrahts in Abstand voneinander reibungsmäßig an. Obwohl hierdurch ein relativ sicheres Festhalten eines Führungsdrahtes an dem Führungsdrahthalter ermöglicht ist, besteht jedoch der Wunsch nach einem gegenüber diesem bekannten Führungsdrahthalter einfacheren Halteraufbau.

Ferner gehört zu der erstgenannten Lösung ein Führungsdrahthalter (US 2006/195117 A1; WO 2007/086876 A2), der ein Führungsdraht-Umlenkteil und ein daran angebrachtes Führungsdraht-Sicherungsteil aufweist, das mit Vorsprüngen und/oder Nuten versehen ist. Diese Vorsprünge und/oder Nuten sind wie bei dem zuvor betrachteten bekannten Führungsdrahthalter in einer Mehrzahl, zumindest dreifach vorgesehen. Obwohl auch hierdurch ein relativ sicheres Festhalten eines Führungsdrahtes an dem Führungsdrahthalter ermöglicht ist, besteht jedoch auch in diesem Fall der Wunsch nach einem gegenüber diesem bekannten Führungsdrahthalter einfacheren Halteraufbau.

Zu der oben erwähnten anderen Lösung gehört eine Führungsdraht- und Katheterverriegelungsvorrichtung (US 2004/0162465 A1; US 7 060 052 B2; EP 1 079 883 B1; DE 699 26 124 T2), die mit einer Anbringungseinrichtung an einem Endoskop anbringbar ist und die in einem sich an die Anbringungseinrichtung anschließenden im Wesentlichen steifen Körperteil eine Einführöffnung zum Einführen eines Führungsdrahtes und einen in der betreffenden Einführöffnung gebildeten Verriegelungsschlitz zum Einklemmen des Führungsdrahtes aufweist. Diese Art der Sicherung des Führungsdrahtes gegenüber seinem Verschieben genügt jedoch zuweilen nicht. Außerdem kann es vorkommen, dass die Außenseite des Führungsdrahtes infolge einer wenn auch geringfügigen Verschiebung in der Einklemmposition durch eine übermäßig starke Reibung an den Kanten des Verriegelungsschlitzes beschädigt wird. Ein derartig beschädigter Führungsdraht ist dann für eine weitere Verwendung nicht mehr brauchbar und daher nicht wieder verwendbar.

Der Erfindung liegt daher die Aufgabe zu Grunde, einen Weg zu zeigen, wie ein Führungsdrahthalter der eingangs genannten Art weiterzubilden ist, damit ein einfacherer Halteraufbau als bei den beiden eingangs betrachteten bekannten Führungsdrahthaltern und zugleich auch eine sichere Aufnahme eines Führungsdrahtes sichergestellt sind. Gelöst wird die vorstehend aufgezeigte Aufgabe durch einen erfindungsgemäßen Führungsdrahthalter mit den Merkmalen des Anspruchs 1.

Die Erfindung wendet zum Festhalten eines Führungsdrahtes an einem Führungsdrahtträger somit ein anderes Prinzip zur Ausübung einer Reibwirkung auf den Führungsdraht an als die oben betrachteten beiden bekannten Festhalteprinzipien. Gemäß der Erfindung ist eine Reibwirkung auf den Führungsdraht durch die für dessen Aufnahme auf dem betreffenden Führungsdrahtträger vorgesehene Führungsbahn ausübbar.

Die Erfindung bringt den Vorteil mit sich, dass auf relativ einfache Weise sowohl ein einfacherer Halteraufbau als bei den beiden eingangs betrachteten bekannten Führungsdrahthaltern als auch eine sichere Aufnahme eines Führungsdrahtes erreicht sind. Überdies lässt sich der Führungsdraht in seinem von einer Verbindungsstelle an dem Anbringungsteil entfernt liegenden Ende in vorteilhafter Weise zusätzlich unter Ausnutzung des Abkröpfungsbereiches klemmen und in eine gewünschte Richtung von dem Führungsdrahtträger und damit von dem Führungsdrahthalter weg leiten und somit gewissermaßen "aufräumen".

Vorzugsweise enthält die Führungsbahn zwischen dem Führungsdraht-Umlenkglied und der genannten einen Führungsdraht-Anlagefläche eine weitere Führungsdraht-Anlagefläche an oder nahe der von dem Anbringungsteil abgewandten Seite des Führungsdrahtträgers, und durch diese weitere Führungsdraht-Anlagefläche ist ein weiterer Reibungswiderstand gegenüber dem über die genannte Seite des Führungsdrahtträgers geführten Führungsdraht ausübbar. Diese Maßnahme bringt den Vorteil mit sich, dass der auf den Führungsdraht an oder nahe der genannten Seite des Führungsdrahtträgers ausübbare Reibungswiderstand noch weiter erhöht werden kann.

Zweckmäßigerweise ist die Führungsbahn des Führungsdrahtes durch einen unterhalb der genannten Seite des Führungsdrahtträgers liegenden Trägerbereich gebildet, in welchem der Führungsdraht von der einen Führungsdraht-Anlagefläche und der weiteren Führungsdraht-Anlagefläche aufnehmbar ist. Hierdurch ergibt sich der Vorteil einer besonders sicheren Führung des Führungsdrahtes an oder nahe der genannten Seite des Führungsdrahtträgers.

Gemäß weiterer zweckmäßiger Ausgestaltung der Erfindung ist die weitere Führungsdraht-Anlagefläche aufgeraut. Dadurch ergibt sich der Vorteil eines verstärkten Reibungswiderstands gegenüber dem über die bzw. auf der von dem Anbringungsteil abgewandten Seite des Führungsdrahtträgers geführten Führungsdraht.

Gemäß noch weiterer zweckmäßiger Ausgestaltung der Erfindung ist die genannte eine Führungsdraht-Anlagefläche durch eine in dem Führungsdrahtträger enthaltene Rolleneinrichtung gebildet. Diese in vorteilhafter Weise zur Richtungsumkehr der Führungsbahn des Führungsdrahtes heranziehbare Rolleneinrichtung ist vorzugsweise eine feststehende Rolleneinrichtung.

Nach einer anderen zweckmäßigen Weiterbildung der Erfindung ist die Rolleneinrichtung durch zwei federnd zusammengeführte Rolleneinrichtungsteile gebildet, zwischen denen der Führungsdraht aufnehmbar ist. Hierdurch ergibt sich der Vorteil, dass auf den Führungsdraht durch die Rolleneinrichtung eine relativ starke Reibungskraft ausgeübt werden kann, die durch entsprechende Festlegung der die Rolleneinrichtungsteile zusammenführenden Federkraft unterschiedlichen Bedürfnissen hinsichtlich der auf den Führungsdraht auszuübenden Klemmkraft angepasst werden kann.

Zweckmäßigerweise ist jede der vorstehend genannten Rolleneinrichtungen durch ein einzelnes Rollenglied gebildet. Dies bringt den Vorteil einer besonders einfachen Rollenkonstruktion mit sich.

Zweckmäßigerweise weist der Führungsdrahtträger bei Betrachtung von seiner einen Seite einen S-förmigen Verlauf auf. Hierdurch ergibt sich der Vorteil, dass der Führungsbahn für den Führungsdraht ein besonders günstiger Verlauf hinsichtlich der Erzielung einer ausgezeichneten Klemmwirkung auf den Führungsdraht gegeben werden kann.

An Hand von Zeichnungen wird die Erfindung nachstehend an einem Ausführungsbeispiel näher erläutert.

In den Zeichnungen zeigen
Fig. 1 eine schematische Seitenansicht eines Führungsdrahthalters gemäß einer Ausführungsform der Erfindung in einer Größe, die von der in der Praxis verwendeten Größe verschieden sein kann,
Fig. 2 eine Draufsicht auf den in Fig. 1 gezeigten Führungsdrahthalter gemäß einer Ausführungsform der Erfindung und
Fig. 3 eine Perspektivansicht des in Fig. 1 und 2 gezeigten Führungsdrahthalters gemäß einer Ausführungsform der Erfindung.

Bevor auf die Zeichnungen näher eingegangen wird, sei angemerkt, dass in sämtlichen Zeichnungen gleiche oder einander entsprechende Einrichtungen bzw. Elemente mit gleichen Bezugszeichen bezeichnet sind.

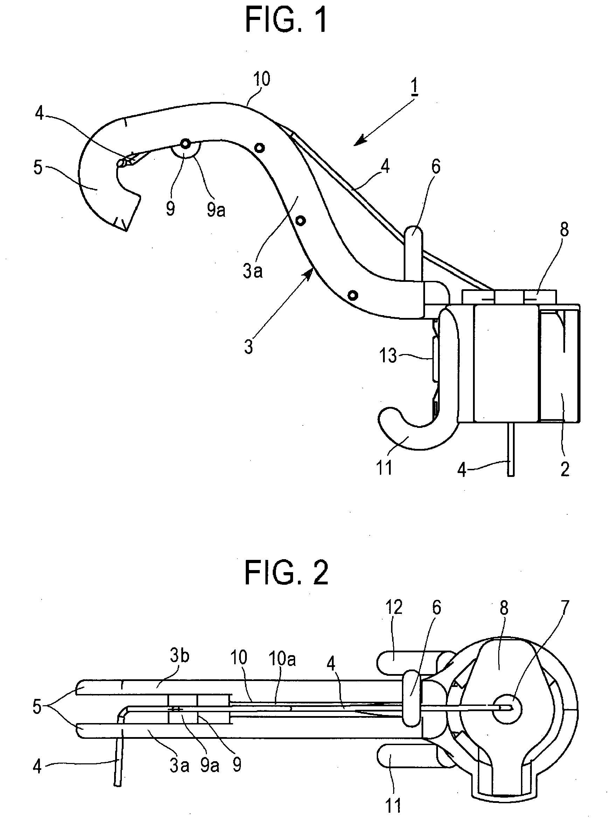

In Fig. 1 ist ein Führungsdrahthalter 1 gemäß einem Ausführungsbeispiel in einer Seitenansicht dargestellt. Dieser Führungsdrahthalter 1 enthält ein Anbringungsteil 2 zum Anbringen des Führungsdrahthalters 1 an einem (hier nicht dargestellten) medizinischen Gerät, welches insbesondere ein Endoskop sein kann, an dessen Arbeitskanalanschluss das betreffende Anbringungsteil 2 angebracht werden kann. Anstelle eines Endoskops kann jedoch gegebenenfalls ein anderes medizinisches Führungsdraht-Einführgerät in Betracht kommen. Das Anbringungsteil 2 kann zum Beispiel ein Aufsetzteil oder eine Kappe sein, die auf ein Aufnahmeteil des jeweiligen medizinischen Geräts aufsetzbar ist und dort nicht leicht abziehbar ist. Das betreffende Anbringungsteil 2 kann aber gegebenenfalls auch ein Schraubverbindungsteil enthalten, mit dem es auf oder in einen entsprechenden Schraubverbindungsteil des jeweiligen medizinischen Geräts verschraubbar ist.

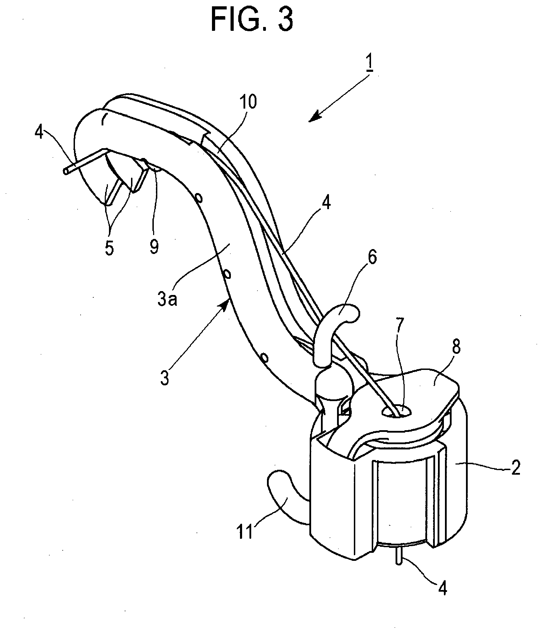

Mit dem Anbringungsteil 2 ist ein Führungsdrahtträger 3 verbunden, der einen Führungsdraht 4 mittels einer Führungsdraht-Aufnahmeeinrichtung festzuhalten gestattet. Der Führungsdrahtträger 3 enthält, wie dies aus den Fig. 2 und 3 deutlich ersichtlich ist, zwei miteinander verbundene Trägerarme 3a und 3b. Die Verbindung der beiden Trägerarme 3a und 3b ist in Fig. 1 und 3 durch kleine Kreise an dem Trägerarm 3a angedeutet. Die beiden Trägerarme 3a und 3b können jedoch vorzugsweise Glieder eines einzigen Formteiles sein, zu dem gegebenenfalls noch weitere Elemente des Führungsdrahthalters 1 gehören können, wie dies weiter unten noch näher ersichtlich werden wird.

Der Führungsdrahtträger 3 und damit dessen beide Trägerarme 3a und 3b weisen bei Betrachtung von der einen Seite des Führungsdrahtträgers 3 - das heißt in Fig. 1 von der Rückseite des Führungsdrahthalters 1 aus - einen S-förmigen Verlauf auf. Entsprechend diesem S-förmigen Verlauf steigen die beiden Trägerarme 3a und 3b von dem Anbringungsteil 2 nach oben, um nach Erreichen einer bestimmten festgelegten Höhe wieder abzufallen und schließlich an ihrem jeweiligen Ende in einem Abkröpfungsbereich 5 auszulaufen. In diesem Abkröpfungsbereich 5 ist der Bereich zwischen den beiden Trägerarmen 3a und 3b ab einem weiter unten noch näher zu betrachtenden Führungsdraht-Umlenkelement 9 offen, um den Führungsdraht 4 von dem Kröpfungsteil eines der Trägerarme 3a und 3b aufnehmen zu lassen.

Die Führungsdraht-Aufnahmeeinrichtung des Führungsdrahtträgers 3 gestattet es, den Führungsdraht 4 festzuhalten, so dass dieser nicht in seiner Längsrichtung verschoben bzw. gezogen werden kann. Dazu umfasst die Führungsdraht-Aufnahmeeinrichtung zum einen ein auf einem flachen Anfangsbereich des Führungsdrahtträgers 3 sich erhebendes Führungsdraht-Umlenkglied 6, das als einseitig offener Umlenkbügel oder Spannbügel ausgebildet ist. Dieses Führungsdraht-Umlenkglied 6 leitet den Führungsdraht 4 in Abstand von dem Anbringungsteil 2 in eine von dessen Durchführungsrichtung in dem Anbringungsteil 2 wegführende Richtung um und spannt dabei den Führungsdraht 4, wie dies aus Fig. 1 ersichtlich ist. Der Führungsdraht 4 wird dadurch aus seiner Durchführungsrichtung in dem Anbringungsteil 2 umgelenkt, und er liegt dabei an einer oberen Kante einer Durchgangsöffnung 7 einer Abdeckplatte 8 des Anbringungsteiles 2 an. An dieser Anlagestelle wird somit auf den Führungsdraht 4 eine Reibungswirkung ausgeübt.

Zum anderen umfasst die Führungsdraht-Aufnahmeeinrichtung in weiterem Abstand von dem Anbringungsteil 2 das bereits erwähnte Führungsdraht-Umlenkelement 9 als zusätzliches Führungsdraht-Umlenkelement, welches mit einer Führungsdraht-Anlagefläche an oder nahe der von dem Anbringungsteil 2 abgewandten Seite des Führungsdrahtträgers 3 gebildet ist, genauer gesagt zwischen den beiden Trägerarmen 3a und 3b des Führungsdrahtträgers 3, die durch dieses Führungsdraht-Umlenkelement 9 miteinander verbunden sind. Auf dieser Führungsdraht-Anlagefläche ist der Führungsdraht 4 längs einer Führungsbahn führbar, durch die im Bereich der betreffenden Führungsdraht-Anlagefläche ein Reibungswiderstand gegenüber dem über die bzw. auf der von dem Anbringungsteil 2 abgewandten Seite des Führungsdrahtträgers 3 geführten Führungsdraht 4 ausübbar ist. Die betreffende Führungsbahn verläuft vorzugsweise, wie dies aus den Fig. 1 bis 3 ersichtlich ist, an oder nahe der genannten Seite des Führungsdrahtträgers 3 und zwischen dessen Trägerarmen 3a und 3b, und zwar etwa in der Mitte zwischen diesen Trägerarmen 3a und 3b.

Das zusätzliche Führungsdraht-Umlenkelement 9 ist bei der vorliegenden Ausführungsform durch eine mit demselben Bezugszeichen 9 versehene, in dem Führungsdrahtträger 3 enthaltene Rolleneinrichtung gebildet, die hier durch ein einzelnes Rollenglied 9 gebildet ist. Dieses Rollenglied 9 kann ein feststehendes Rollenglied oder ein Rollenglied sein, welches nur schwer drehbar ist, wenn der Führungsdraht 4 über dieses Rollenglied gezogen wird. In beiden Fällen kann das jeweilige Rollenglied 9 eine raue Oberfläche besitzen, so dass dem über dieses Rollenglied 9 gezogene Führungsdraht 4 ein Reibungswiderstand entgegengesetzt wird.

Alternativ kann die Rolleneinrichtung 9 durch zwei federnd zusammengeführte Rolleneinrichtungsteile gebildet sein, zwischen denen der Führungsdraht 4 aufnehmbar ist. Auch in diesem Fall können die beiden Rolleneinrichtungsteile jeweils eine raue Oberfläche besitzen, so dass auch hierbei dem über dieses Rollenglied 9 gezogene Führungsdraht 4 ein Reibungswiderstand entgegengesetzt wird.

Zusätzlich zu der erläuterten Führungsbahn zwischen dem Führungsdraht-Umlenkglied 6 und dem genannten zusätzlichen Führungsdraht-Umlenkglied 9 mit seiner Anlagefläche 9a ist hier ein weiteres Führungsdraht-Umlenkglied 10 mit einer Anlagefläche 10a an oder nahe der von dem Anbringungsteil 2 abgewandten Seite der hier miteinander verbundenen Trägerarme 3a und 3b des Führungsdrahtträgers 3 vorgesehen, wie dies aus Fig. 2 und 3 näher ersichtlich ist. Durch diese weitere Führungsdraht-Anlagefläche 10a, die sich auf dem höchsten bogenförmigen oberen Abschnitt des Führungsdrahtträgers 3 befindet und die vorzugsweise ebenfalls aufgeraut ist, ist ein weiterer Reibungswiderstand gegenüber dem über die genannte Seite des Führungsdrahtträgers 3 geführten Führungsdraht 4 ausübbar. Der betreffende bogenförmige obere Abschnitt (10) des Führungsdrahtträgers 3 stellt eine Erhöhung einer direkt zwischen dem Führungsdraht-Umlenkglied 6 und der Führungsdraht-Anlagefläche 9a verlaufenden Verbindungslinie dar.

Die Rauigkeit der zuvor erwähnten Führungsdraht-Anlageflächen 9a und 10a kann beispielsweise durch eine Strukturierung dieser Flächen und/oder durch einen auf die betreffenden Flächen aufgebrachten entsprechend rauen Flächenüberzug erreicht sein.

Die erwähnte Führungsbahn des Führungsdrahtes 4 ist hier durch einen unterhalb der genannten Seite des Führungsdrahtträgers 3 liegenden Trägerbereich gebildet, in welchem der Führungsdraht 4 von der einen Führungsdraht-Anlagefläche 9a und der weiteren Führungsdraht-Anlagefläche 10a aufnehmbar ist. Die Absenkung der Führungsbahn von der genannten Seite des Führungsdrahtträgers 3 liegt beispielsweise zwischen 0,5 mm und 4 mm.

Zusätzlich zu den vorstehend beschriebenen Elementen weist der in den Fig. 1 bis 3 dargestellte Führungsdrahthalter 1 noch zwei Haken 11, 12 auf, die zusammen mit dem Führungsdrahtträger 3 an dem Anbringungsteil 2 unterhalb einer Öffnung 13 des Anbringungsteiles 2 angebracht sind, welche mit dem Durchgang des Anbringungsteiles 2 für den Führungsdraht 4 verbunden ist. An die beiden Haken 11 , 12 ist ein (nicht dargestellter) Auffangbeutel anhängbar, mit dem Flüssigkeit aufgefangen werden kann, die aus der genannten Öffnung 13 auszutreten vermag, wenn der Führungsdraht 4 nach dem Einführen in eine Öffnung eines Individuums wieder aus dieser Öffnung herausgezogen wird.

Zum Arbeiten mit dem Führungsdraht 4 in Verbindung mit einem (nicht dargestellten) medizinischen Gerät, wie einem Endoskop, wird der Führungsdraht 4 zunächst durch die Öffnung 7 der Abdeckplatte 8 des auf dem betreffenden medizinischen Gerät aufgesetzten Anbringungsteiles 2 soweit eingeführt, bis die (nicht gezeigte) Spitze des Führungsdrahtes 4 in ein gewünschtes Zielgebiet vorgeschoben worden ist. Sodann wird der aus der Öffnung 7 der Abdeckplatte 8 des Anbringungsteiles 2 herausragende Abschnitt des Führungsdrahtes 4 nach Herunterführen unter das Führungsdraht-Umlenkglied 6 in die Führungsbahn an oder nahe der von dem Anbringungsteil 2 abgewandten Seite des Führungsdrahtträgers 3 aufgelegt, um mit der genannten einen Führungsdraht-Anlagefläche 9a und der weiteren Führungsdraht-Anlagefläche 10a in Anlage zu gelangen. Anschließend wird der von diesen Anlageflächen 9a und 10a abliegende und damit von der Öffnung 7 der Abdeckplatte 8 des Anbringungsteiles 2 am weitesten weg liegende Teil des Führungsdrahtes 4 um den Abkröpfungsbereich 5 des Führungsdrahtträgers 3 herum gelegt, und zwar um einen der Trägerarme 3a und 3b des Führungsdrahtträgers 3 - hier um den Trägerarm 3a. Dadurch wird der Führungsdraht 4 winklig, und zwar um etwa 90° aus seiner Führungsbahn an oder nahe der genannten Seite des Führungsdrahtträgers 3 umgelenkt, wie dies aus Fig. 2 ersichtlich ist. Der Führungsdraht 4 ist damit in eine gewünschte Richtung gewissermaßen "aufgeräumt", in der er in seiner Längsrichtung auf dem Führungsdrahtträger 3 jetzt nicht mehr verschiebbar ist.

Für diese Nichtverschiebbarkeit des Führungsdrahtes 4 in dessen Längsrichtung auf dem Führungsdrahtträger 3 sind vor allem die Anlagen des Führungsdrahtes 4 an der Austrittsstelle aus der Abdeckplatte 8 des Anbringungsteiles 2, an dem Führungsdraht-Umlenkglied 6 auf bzw. an der von dem Anbringungsteil 2 abgewandten Seite des Führungsdrahtträgers 3 und an der Führungsdraht-Anlagefläche 9a an oder nahe der genannten Seite des Führungsdrahtträgers 3 maßgebend. Die Anlagen des Führungsdrahtes 4 an der weiteren Führungsdraht-Anlagefläche 10a und an dem Abkröpfungsbereich 5 des Führungsdrahtträgers 3 wirken sich unterstützend auf die zuvor erläuterte Nichtverschiebbarkeit des Führungsdrahtes 4 in dessen Längsrichtung auf dem Führungsdrahtträger 3 aus.

Abschließend sei noch angemerkt, dass das Anbringungsteil 2 aus einem biokompatiblen Material, wie einem Metall oder Kunststoff, zum Beispiel aus einem Gummimaterial bestehen kann. Alle übrigen Elemente des hier beschriebenen Führungsdrahthalters 1 bestehen ebenfalls aus einem biokompatiblen Material, wie aus einem Metall oder Kunststoff, wie zum Beispiel aus einem ABS-Kunststoff (Acrylnitril-Butadien-StyrolCopolymerisat), und zwar vorzugsweise als ein einziges, zusammenhängendes Form- bzw. Spritzgussteil. Dabei sind Kanten von Teilen des Führungsdrahthalters 1, an denen der Führungsdraht 4 entlang geführt ist, vorzugsweise verrundet, um eine Beschädigung der Führungsdrahtoberfläche zu vermeiden und um ein Einlegen des Führungsdrahtes 4 in den Führungsdrahthalter 1 für den Anwender zu vereinfachen.

Der Führungsdraht 4 besteht, wie an sich bekannt, aus einem Edelstahlkern, der mittels Polytetrafluorethylen - PTFE - (Handelsmarke: Teflon) ummantelt ist.

### Bezugszeichenliste

- 1: Führungsdrahthalter
- 2: Anbringungsteil
- 3: Führungsdrahtträger
- 3a: Trägerarm
- 3b: Trägerarm
- 4: Führungsdraht
- 5: Abkröpfungsbereich
- 6: Umlenkglied
- 7: Durchgangsöffnung
- 8: Abdeckplatte
- 9: Führungsdraht-Umlenkelement, Rolleneinrichtung, Rolle
- 9a: Führungsdraht-Anlagefläche
- 10: Führungsdraht-Umlenkelement
- 10a: Führungsdraht-Anlagefläche
- 11: Haken
- 12: Haken
- 13: Öffnung

## Patentansprüche

1. Führungsdrahthalter (1) zum Aufnehmen und Festhalten eines medizinischen Führungsdrahtes (4) und zum Anbringen an einem medizinischen Gerät, insbesondere an einem Endoskop, mit einem Anbringungsteil (2), durch welches der Führungsdraht (4) hindurchführbar ist, und einem mit dem Anbringungsteil (2) verbundenen und von diesem abstehenden Führungsdrahtträger (3), der den Führungsdraht (4) mittels einer Führungsdraht-Aufnahmeeinrichtung (5, 6, 9) festzuhalten gestattet, die ein erstes, den Führungsdraht (4) in Abstand von dem Anbringungsteil (2) in eine von seiner Durchführungsrichtung in dem Anbringungsteil (2) wegführende Richtung umleitendes und spannendes Führungsdraht-Umlenkglied (6) enthält, an das sich in weiteren Abständen von dem Anbringungsteil (2) ein zusätzliches Führungsdraht-Umlenkelement (9) und ein noch weiteres Führungsdraht-Umlenkelement (5) anschließen,
**dadurch gekennzeichnet,**
**dass** der Führungsdrahtträger (3) zwei miteinander verbundene Trägerarme (3a, 3b) enthält,
**dass** die Führungsdraht-Aufnahmeeinrichtung des Führungsdrahtträgers (3) als erstes Führungsdraht-Umlenkglied (6) einen auf einem flachen Anfangsbereich sich erhebenden, einseitig offenen Umlenk- oder Spannbügel (6) umfasst, der den Führungsdraht (4) in Abstand von dem Anbringungsteil (2) in eine von dessen Durchführungsrichtung in dem Anbringungsteil (2) wegführende Richtung umzuleiten und dabei den Führungsdraht (4) zu spannen gestattet,
**dass** die Führungsdraht-Aufnahmeeinrichtung des Führungsdrahtträgers (3) in weiterem Abstand von dem Anbringungsteil (2) als zusätzliches Führungsdraht-Umlenkelement (9) eine Führungsdraht-Anlagefläche (9a) zwischen den beiden Trägerarmen (3a, 3b) an oder nahe der von dem Anbringungsteil (2) abgewandten Seite des Führungsdrahtträgers (3) umfasst, auf der der Führungsdraht (4) längs einer Führungsbahn (bei 9a) führbar ist, durch die im Bereich der betreffenden Führungsdraht-Anlagefläche (9a) ein Reibungswiderstand gegenüber dem über die genannte Seite des Führungsdrahtträgers (3) geführten Führungsdraht (4) ausübbar ist,
und **dass** die beiden Trägerarme (3a, 3b) des Führungsdrahtträgers (3) an ihren von einer Verbindungsstelle an dem Anbringungsteil (2) entfernt liegenden Enden als noch weiteres Führungsdraht-Umlenkelement (5) jeweils einen offenen Abkröpfungsbereich (5) aufweisen, in welchem der Führungsdraht (4) winklig aus seiner Führungsbahn an oder nahe der genannten Seite des Führungsdrahtträgers (3) um einen der Trägerarme (3a, 3b) umlenkbar ist.

2. Führungsdrahthalter nach Anspruch 1,
**dadurch gekennzeichnet, dass** die Führungsbahn zwischen dem Führungsdraht-Umlenkglied (6) und der genannten einen Führungsdraht-Anlagefläche (9a) des zusätzlichen Führungsdraht-Umlenkelements (9) eine weitere Führungsdraht-Anlagefläche (10a) an oder nahe der genannten Seite des Führungsdrahtträgers (3) enthält und dass durch diese weitere Führungsdraht-Anlagefläche (10a) ein weiterer Reibungswiderstand gegenüber dem über die genannte Seite des Führungsdrahtträgers (3) geführten Führungsdraht (4) ausübbar ist.

3. Führungsdrahthalter nach Anspruch 2,
**dadurch gekennzeichnet, dass** die Führungsbahn des Führungsdrahtes (4) durch einen von der genannten Seite des Führungsdrahtträgers (3) aus abgesenkten Trägerbereich gebildet ist, in welchem der Führungsdraht (4) von der einen Führungsdraht-Anlagefläche (9a) und der weiteren Führungsdraht-Anlagefläche (10a) aufnehmbar ist.

4. Führungsdrahthalter nach Anspruch 2 oder 3,
**dadurch gekennzeichnet, dass** die weitere Führungsdraht-Anlagefläche (10a) aufgeraut ist.

5. Führungsdrahthalter nach einem der Ansprüche 1 bis 4,
**dadurch gekennzeichnet, dass** die genannte eine Führungsdraht-Anlagefläche (9a) auf einer in dem Führungsdrahtträger (3) enthaltene Rolleneinrichtung (9) gebildet ist.

6. Führungsdrahthalter nach Anspruch 5,
**dadurch gekennzeichnet, dass** die Rolleneinrichtung (9) durch zwei federnd zusammengeführte Rolleneinrichtungsteile gebildet ist, zwischen denen der Führungsdraht (4) aufnehmbar ist.

7. Führungsdrahthalter nach Anspruch 5 oder 6,
**dadurch gekennzeichnet, dass** die Rolleneinrichtung (9) durch ein einzelnes Rollenglied (9) gebildet ist.

8. Führungsdrahthalter nach einem der Ansprüche 1 bis 7,
**dadurch gekennzeichnet, dass**
der Führungsdrahtträger (3) bei Betrachtung von seiner einen Seite einen S-förmigen Verlauf aufweist.

## Claims

1. A guide wire holder (1) for accommodating and holding a medical guide wire (4) in place and for being affixed to a medical device, in particular on an endoscope, the guide wire holder (1) comprising an affixation part (2), through which the guide wire (4) is capable of being passed, and a guide wire carrier (3) connected with the affixation part (2) and projecting away from same, the guide wire carrier (3) allowing for holding the guide wire (4) in place by a guide wire accommodation device (5, 6, 9), which comprises a first guide wire deflection element (6) deflecting and tensing the guide wire (4) at a distance from the affixation part (2) in a direction leading away from its pass-through direction in the affixation part (2), from which guide wire deflection element (6) at further distances from the affixation part (2) extend an additional guide wire deflection element (9) and a yet further guide wire deflection element (5),
**characterized in that**
the guide wire carrier (3) contains two carrier arms (3a, 3b) connected with one another,
the guide wire accommodation device of the guide wire carrier (3) as first guide wire deflection element (6) comprises a deflection or tensing bracket (6) rising on a flat initial region and being open on one side, which allows for deflecting the guide wire (4) at a distance from the affixation part (2) in a direction leading away from its pass-through direction in the affixation part (2) and in this connection for tensing the guide wire (4),
the guide wire accommodation device of the guide wire carrier (3) at a further distance from the affixation part (2) as additional guide wire deflection element (9) comprises a guide wire contact surface (9a) between the two carrier arms (3a, 3b) on or close to the side of the guide wire carrier (3) facing away from the affixation part (2), on which the guide wire (4) is guidable along a guide track (at 9a), by which in the region of the respective guide wire contact surface (9a) a friction resistance is capable of being exerted on the guide wire (4) guided over the named side of the guide wire carrier (3),
and **in that** the two carrier arms (3a, 3b) of the guide wire carrier (3) on their ends that lie farthest from a contact location on the affixation part (2) as yet further guide wire deflection element (5) each have an open offset region (5), in which the guide wire (4) is deflectable at an angle out of its guide track on or close to the named side of the guide wire carrier (3) around one of the carrier arms (3a, 3b).

2. The guide wire holder according to claim 1,
**characterized in that** the guide track between the guide wire deflection element (6) and the named one guide wire contact surface (9a) of the additional guide wire deflection element (9) contains a further guide wire contact surface (10a) on or close to the named side of the guide wire carrier (3) and that by this further guide wire contact surface (10a) a further friction resistance is capable of being exerted on the guide wire (4) guided over the named side of the guide wire carrier (3).

3. The guide wire holder according to claim 2,
**characterized in that** the guide track of the guide wire (4) is formed by a carrier region which lies below the named side of the guide wire carrier (3) and in which the guide wire (4) is capable of being accommodated by the one guide wire contact surface (9a) and the further guide wire contact surface (10a).

4. The guide wire holder according to claim 2 or 3,
**characterized in that** the further guide wire contact surface (10a) is roughened.

5. The guide wire holder according to any one of claims 1 to 4,
**characterized in that** the named one guide wire contact surface (9a) is formed on a roller device (9) contained in the guide wire carrier (3).

6. The guide wire holder according to claim 5,
**characterized in that** the roller device (9) is formed by two roller device parts brought together in a resilient manner, between which the guide wire (4) is capable of being accommodated.

7. The guide wire holder according to claim 5 or 6,
**characterized in that** the roller device (9) is formed by a single roller element (9).

8. The guide wire holder according to any one of claims 1 to 7,
**characterized in that** the guide wire carrier (3), when looked at from its one side, has an S-shaped progression.

## Revendications

1. Porte-câble de guidage (1) pour la réception et le maintien ferme d'un câble de guidage médical (4) et pour la pose sur un appareil médical, en particulier sur un endoscope, comportant une partie pour la pose (2), à travers laquelle le câble de guidage (4) peut être passé, et un support (3) de câble de guidage relié à la partie pour la pose (2) et faisant saillie à partir de celle-ci, qui permet au câble de guidage (4) de se maintenir fermement au moyen d'un dispositif de réception de câble de guidage (5, 6, 9) qui contient un premier élément déflecteur (6) de câble de guidage déviant et étirant le câble de guidage (4) à distance de la partie pour la pose (2) dans une direction partant de sa direction de mise en œuvre dans la partie pour la pose (2), auquel se raccorde un élément déflecteur (9) de câble de guidage supplémentaire et encore un autre élément déflecteur (5) de câble de guidage à d'autres distances de la partie pour la pose (2),
**caractérisé en ce**
**que** le support (3) de câble de guidage contient deux bras de support (3a, 3b) reliés entre eux,
**que** le dispositif de réception de câble de guidage du support du support (3) de câble de guidage comporte un étrier déflecteur ou de tension (6) ouvert d'un côté s'élevant sur une zone initiale plane comme premier élément déflecteur (6) de câble de guidage, qui permet de dévier le câble de guidage (4) à distance de la partie pour la pose (2) dans une direction partant de sa direction de mise en œuvre dans la partie pour la pose (2) et d'étirer le câble de guidage (4) dans ce processus, que le dispositif de réception de câble de guidage du support (3) de câble de guidage à une distance ultérieure de la partie pour la pose (2) comme élément déflecteur de câble (9) de guidage supplémentaire comporte une surface d'appui de câble de guidage (9a) entre les deux bras de support (3a, 3b) au côté ou près du coté du support (3) de câble de guidage opposé de la partie pour la pose (2) sur lequel le câble de guidage (4) peut être guidé le long d'un trajet de guidage (près de 9a), au moyen duquel dans la zone de la surface d'appui de câble de guidage (9a) concernée une résistance au frottement peut être exercée par rapport au câble de guidage (4) guidé sur ledit côté du support (3) de câble de guidage,
et **que** les deux bras de support (3a, 3b) du support (3) de câble de guidage sur leurs extrémités éloignées d'un point de raccordement sur la partie pour la pose (2) comme un élément déflecteur (5) de câble de guidage ultérieur chacun comporte une partie ouverte coudée (5) dans laquelle le câble de guidage (4) audit ou près dudit coté du support (3) de câble de guidage peut être dévié angulairement de son trajet de guidage autour d'un de ses bras de support (3a, 3b).

2. Porte-câble de guidage selon la revendication 1,
**caractérisé en ce que** le trajet de guidage entre l'élément déflecteur (6) de câble de guidage et ladite une surface d'appui de câble de guidage (9a) de élément déflecteur (9) de câble de guidage supplémentaire comporte une autre surface d'appui de câble de guidage (10a) audit ou près dudit côté du support (3) de câble de guidage et que par cette autre surface d'appui de câble de guidage (10a) un autre résistance au frottement peut être exercée par rapport au câble de guidage (4) guidé sur ledit côté du support (3) de câble de guidage.

3. Porte-câble de guidage selon la revendication 2,
**caractérisé en ce que** le trajet de guidage du câble de guidage (4) est formé par une zone de support abaissée dudit côté du support (3) de câble de guidage, dans laquelle le câble de guidage (4) peut être reçu par l'une surface d'appui de câble de guidage (9a) et l'autre surface d'appui de câble de guidage (10a).

4. Porte-câble de guidage selon la revendication 2 ou 3,
**caractérisé en ce que** l'autre surface d'appui de câble de guidage (10a) est rugueuse.

5. Porte-câble de guidage selon l'une quelconque des revendications 1 à 4,
**caractérisé en ce que** ladite une surface d'appui de câble de guidage (9a) est formée sur un dispositif de rouleau (9) contenu dans le support (3) de câble de guidage.

6. Porte-câble de guidage selon la revendication 5,
**caractérisé en ce que** le dispositif de rouleau (9) est formé par deux parties de moyen de rouleau réunies de manière résiliente entre lesquelles le câble de guidage (4) peut être reçu.

7. Porte-câble de guidage selon la revendication 5 ou 6,
**caractérisé en ce que** le dispositif de rouleau (9) est formé par un seul élément de rouleau.

8. Porte-câble de guidage selon l'une quelconque des revendications 1 à 7, **caractérisé en ce que**
le support (3) de câble de guidage vu de l'un de ses côtés comporte un tracé en forme de S.
